# EUROPEAN PATENT APPLICATION

(11) **EP 2 891 465 A1**
(43) Date of publication of application: **08.07.2015**
(21) Application number: 13832228.4
(22) Date of filing: 30.08.2013
(51) Int. Cl.: A61B 19/00, A61B 1/00, A61B 17/28

(54) **MEDICAL SYSTEM AND OPERATION METHOD**

(30) Priority: 30.08.2012 JP 2012189881
(71) Applicant: OLYMPUS CORPORATION, Shibuya-ku Tokyo 151-0072 (JP)
(72) Inventor: ISODA, Takumi, Tokyo 151-0072 (JP)
(74) Representative: Gunzelmann, Rainer
(86) International application number: PCT/JP2013/073274
(87) International publication number: WO 2014/034837

(57) **Abstract**

The present medical system has an external tube, a treatment tool channel, a treatment tool inserted into the treatment tool channel, and a holding tool provided on the external tube, wherein the holding tool has a grip part for holding the treatment tool and a joint for moving the grip part with respect to the external tube, and the treatment tool has a treatment part that performs treatment for a site to be treated, a flexible tube part provided at an end thereof with the treatment part and inserted into the treatment tool channel, and a ring part provided on at least one of the treatment part and the flexible tube part and gripped by the grip part.

## Description

### Technical Field

The present invention relates to a medical system and an operation method.

Priority is claimed on Japanese Patent Application No. 2012-189881, filed August 30, 2012, the content of which is incorporated herein by reference.

### Background Art

Conventionally, a treatment tool used together with an endoscope has been known as a treatment tool for performing treatment in the inside of a body. For example, Patent Literature 1 discloses an endoscope treatment system including an endoscope provided with an insertion part inserted into the inside of the body and a treatment tool mounted in the insertion part of the endoscope.

In the endoscope treatment system disclosed in Patent Literature 1, the treatment tool mounted in the endoscope is detachable from the endoscope and can be received in a receiving part mounted in the endoscope. Furthermore, in Patent Literature 1, forceps are provided as an example of the treatment tool.

### Citation List

### Patent Literature

### Patent Literature 1

Japanese Patent No. 4271088

### Summary of Invention

### Problem to be Solved by Invention

In the endoscope treatment system disclosed in Patent Literature 1, in order to change the direction and orientation of the forceps, it is necessary to change the direction and orientation of the insertion part. Therefore, in the endoscope treatment system disclosed in Patent Literature 1, since there is a limitation in changing the direction and orientation of the insertion part in a narrow lumen, there is a limitation in the direction and orientation of disposal of the treatment tool in a target part and skill is required in operation.

An object of the present invention is to provide a medical system capable of simply changing the direction, position, orientation or the like of a device such as a treatment tool while maintaining the direction of an insertion part, and an operation method of the device such as a treatment tool.

### Means for Solving Problem

A medical system according to a first aspect of the present invention includes an insertion part inserted into an inside of a body, an arm part provided on the insertion part and capable of moving with respect to the insertion part, a device disposed in the inside of the body, a holding part provided on the arm part and holding the device, and a part to be held provided on the device and held by the holding part.

According to a second aspect of the present invention, in the medical system according to the first aspect, at least one of the holding part and the part to be held may have a restriction part that restricts movement of the device.

According to a third aspect of the present invention, in the medical system according to the first aspect or the second aspect, the holding part may have a grip part that grips the part to be held, and the part to be held may have a part to be gripped capable of engaging with the grip part.

According to a fourth aspect of the present invention, in the medical system according to any one of the first aspect to the third aspect, the holding part and the part to be held may have concave-convex shapes engaging with each other.

According to a fifth aspect of the present invention, in the medical system according to any one of the first aspect to the fourth aspect, the holding part and the part to be held may engage with each other through frictional force.

According to a sixth aspect of the present invention, in the medical system according to any one of the first aspect to the fifth aspect, a rod-shaped part may be provided on one of the holding part and the part to be held, and a hole to which the rod-shaped part is fitted may be formed in a remaining one of the holding part and the part to be held.

According to a seventh aspect of the present invention, in the medical system according to any one of the first aspect to the sixth aspect, the part to be held may have a plurality of projection-stripe portions, and the plurality of projection-stripe portions may extend in parallel to one another in a longitudinal axial direction of the device.

According to an eighth aspect of the present invention, in the medical system according to any one of the first aspect to the seventh aspect, the part to be held may have a plurality of conical-shaped portions having apexes capable of coming in contact with the holding part.

According to a ninth aspect of the present invention, in the medical system according to any one of the first aspect to the eighth aspect, the insertion part may have an external tube and a device channel formed in the external tube, and the device may have a flexible tube part insertable into the device channel.

According to a tenth aspect of the present invention, in an operation method of a device disposed in an inside of a body, a part of the device is held using a holding part provided on an arm part that is the body provided separately from the device and has at least one degree of freedom, and the arm part is moved, so that the device held by the holding part is moved together with the arm part.

### Advantageous Effects of Invention

According to the medical system and the operation method of a device disposed in the inside of a body, it is possible to simply change the direction, position, orientation or the like of the device such as a treatment tool while maintaining the direction of the insertion part.

### Brief Description of Drawings

Fig. 1 is an overall view of a medical system according to one embodiment of the present invention.
Fig. 2 is a perspective view showing the structure of a part of a medical system according to one embodiment of the present invention.
Fig. 3 is a schematic diagram showing a treatment tool in a medical system according to one embodiment of the present invention.
Fig. 4 is a diagram showing the operation of a medical system when used according to one embodiment of the present invention.
Fig. 5A is a schematic diagram showing the structure of a modification 1 of one embodiment of the present invention.
Fig. 5B is a schematic diagram showing the structure of a modification 1 of one embodiment of the present invention.
Fig. 6A is a schematic diagram showing the structure of a modification 2 of one embodiment of the present invention.
Fig. 6B is a schematic diagram showing the structure of a modification 2 of one embodiment of the present invention.
Fig. 7 is a schematic diagram showing the structure of a modification 3 of one embodiment of the present invention.
Fig. 8 is a schematic diagram showing the structure of a modification 4 of one embodiment of the present invention.
Fig. 9 is a schematic diagram showing the structure of a modification 5 of one embodiment of the present invention.
Fig. 10 is a schematic diagram showing the structure of a modification 6 of one embodiment of the present invention.
Fig. 11 is a schematic diagram showing the structure of a modification 7 of one embodiment of the present invention.
Fig. 12 is a schematic diagram showing the structure of a modification 8 of one embodiment of the present invention.
Fig. 13 is a schematic diagram showing the structure of a modification 9 of one embodiment of the present invention.
Fig. 14A is a plan view showing an effect in a modification 9 of one embodiment of the present invention.
Fig. 14B is a side view showing an effect in a modification 9 of one embodiment of the present invention.
Fig. 15A is a plan view showing an effect in a modification 9 of one embodiment of the present invention.
Fig. 15B is a side view showing an effect in a modification 9 of one embodiment of the present invention.

### Description of Embodiments

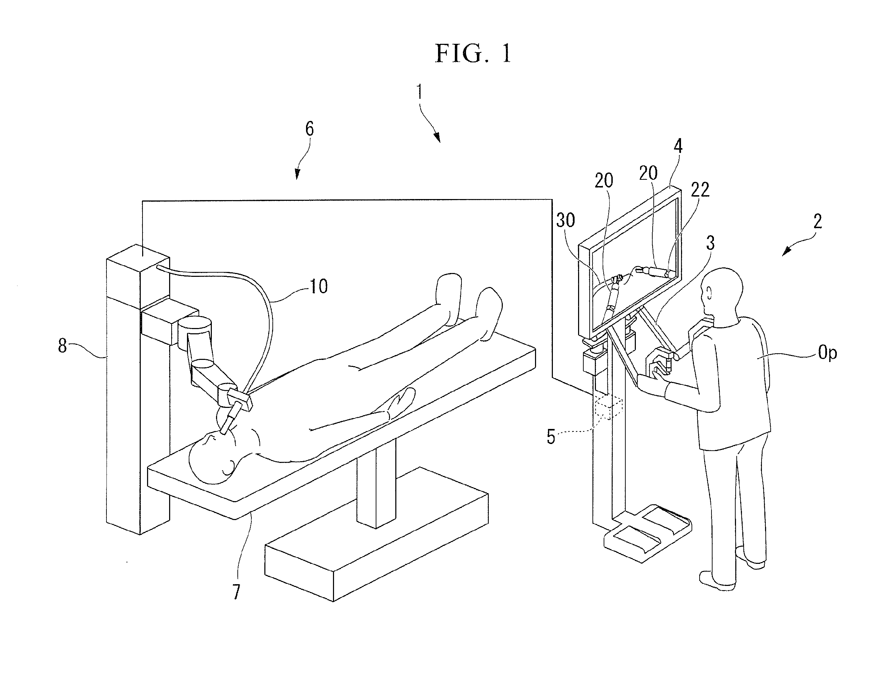

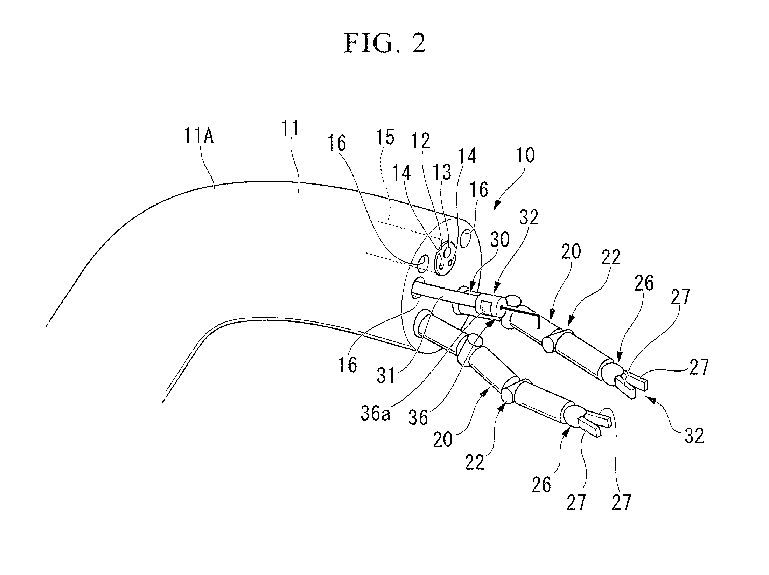

A medical system of one embodiment of the present invention will be described below. Fig. 1 is an overall view of a medical system of the present embodiment. Fig. 2 is a perspective view showing the structure of a part of the medical system.

As shown in Fig. 1 and Fig. 2, a medical system 1 of the present embodiment is a master-slave type system including a master manipulator 2 operated by an operator Op for treatment, and a slave manipulator 6 provided with an endoscope device 10 for treatment.

The master manipulator 2 includes master arms 3, a display part 4, and a control part 5. The master arms 3 are operated by the operator Op. The display part 4 displays video or the like captured using the endoscope device 10 for treatment. The control part 5 generates an operation command for operating the slave manipulator 6 on the basis of the operation of the master arms 3.

In the present embodiment, the master arms 3 are operation parts provided in order to operate holding tools 20 (which will be described later) mounted in the endoscope device 10 for treatment. Although not shown in detail, the master manipulator 2 has the master arms 3 corresponding to the right hand and the left hand of the operator Op. Each of the master arms 3 has a multiple joint structure used to operate a joint part 22 of the holding tool 20 having at least one degree of freedom, which will be described later. A grip part (not shown) for operating a grip part 26 of the holding tool 20, which will be described later, is provided at an end portion of each of the master arms 3 positioned at the operator Op side.

The display part 4 is a device on which the video of a site to be treated, which is captured by an observation device 15 (which will be described later) mounted in the endoscope device 10 for treatment, is displayed. On the display part 4, the video of the holding tool 20 and the video of a treatment tool 30, which will be described later, are also displayed together with the video of the site to be treated.

The slave manipulator 6 has a placement table 7 on which a patient is placed, a multiple-joint robot 8 disposed in the vicinity of the placement table 7, the endoscope device 10 for treatment mounted in the multiple-joint robot 8, the holding tool 20 (an arm part), and the treatment tool 30. The treatment tool 30 is an example of a device disposed inside of a body. The multiple-joint robot 8 operates according to the operation command sent from the master manipulator 2.

As shown in Fig. 2, the endoscope device 10 for treatment has an external tube (an insertion part) and an endoscope channel 12, and the holding tool 20 is mounted at a distal end of the external tube 11.

The external tube 11 of the endoscope device 10 for treatment is a long member that is inserted into the inside of a patient's body. The external tube 11 may be curved by a well-known curved mechanism 11A and can direct the holding tool 20 in a desired direction in the inside of the body. Furthermore, the external tube 11 is provided with a treatment tool channel 16 into which the treatment tool 30 is inserted.

Furthermore, the observation device 15 having an imaging mechanism 13 and an illumination mechanism 14 is inserted into the endoscope channel 12 provided in the external tube 11.

The endoscope channel 12 is provided in the external tube 11 so as to be opened at the distal end of the external tube 11.

The observation device 15 is a device for observing a site to be treated using the imaging mechanism 13 and the illumination mechanism 14. The observation device 15 may also have a curved mechanism (not shown) for directing the imaging mechanism 13 and the illumination mechanism 14 in a desired direction. Furthermore, as the observation device 15, an endoscope having a well-known structure may also be appropriately selected and employed.

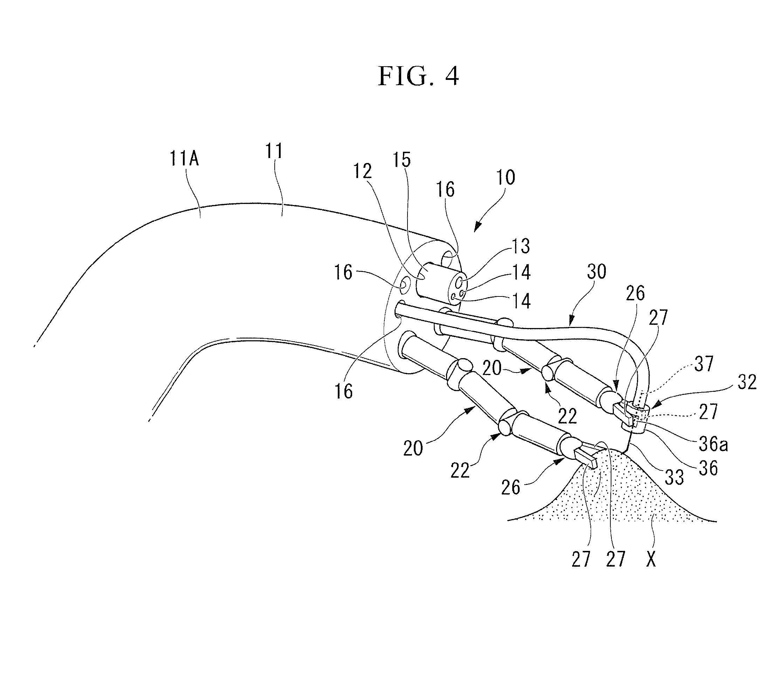

The holding tool 20 has the joint part 22 that is curved by the operation of the master arm 3, and the grip part 26 (a holding part) mounted in the joint part 22. In the holding tool 20, a curvable structure is not limited to the joint structure based on a combination of long tubular parts as shown in Fig. 2 and Fig. 4. For example, in the holding tool 20, as the curvable structure, a joint structure in which a plurality of curved pieces are concatenated to one another so as to rotate may also be employed.

The grip part 26 is a member for holding grooves 36a of a ring part 36, which will be described later, with the grooves 36a interposed therebetween, and has a pair of grip members 27 entering the grooves 36a of the ring part 36. In the grip part 26, the pair of grip members 27 are opened and closed by the operation of the aforementioned operation part. Furthermore, the grip part 26 is further provided with a two-axis joint for bending the grip part 26 at the distal end of the joint part 22.

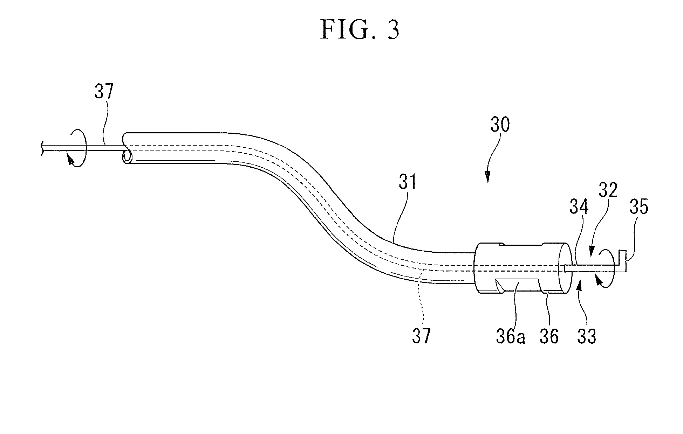

Fig. 3 is a schematic diagram showing the treatment tool 30. As shown in Fig. 3, the treatment tool 30 is a medical device disposed in the inside of a body. The treatment tool 30 has a flexible tube part 31 having flexibility, a treatment part 32, a conducting wire 37, and a plug (not shown). The treatment part 32 is disposed at a first end of the flexible tube part 31. The conducting wire 37 energizes the treatment part 32 with a high frequency current. The plug is connected to the conducting wire 37 disposed at a second end of the flexible tube part 31.

The flexible tube part 31 is a tubular member flexibly formed of a resin and is a long member inserted into the treatment tool channel 16.

The treatment part 32 has a hook-shaped electrode 33, the ring part 36 (a part to be held, a part to be gripped, a restriction part) provided at the distal end of the flexible tube part 31, and is provided in order to perform treatment for a site to be treated. In the present embodiment, the treatment part 32 can incise a biological tissue.

The hook-shaped electrode 33 is a fine line-shaped electrode having a shaft portion 34 extending concentrically with the conducting wire 37, and a hook portion 35 with a protruding end having a shape bent with respect to the shaft portion 34 at 90°.

The ring part 36 is provided at an outer peripheral side of the shaft portion 34 in the hook-shaped electrode 33, and has a pair of grooves 36a. The grooves 36a formed in the ring part 36 are configured to be fitted to the grip members 27. The ring part 36 is made of a material with high biocompatibility. For example, as a metallic ring part 36, stainless steel, a nickel-titanium alloy, a cobalt-chrome-based alloy, pure titanium, a titanium alloy, and a magnesium alloy may be employed. Furthermore, as a bioabsorbable resin ring part 36, PGA, PLA, PDS, TMC, poly ε-caprolacton, and a copolymer thereof may be employed. Furthermore, as the ring part 36 made of a nonbioabsorbable resin, nylon, polyester, polypropylene, polybutester, a fluorine resin or the like may be employed. The ring part 36 has sufficient strength or flexibility so as not to be easily broken such that fragments generated when the ring part 36 is cut or divided by the gripping of the grip members 27 are prevented from remaining in the inside of a body. The ring part 36 is made of a material with high biocompatibility, such that painful of a patient is restricted to a minimum level even though the fragments of the ring part 36 remain inside of the body.

The conducting wire 37 is also used as a wire for advancing, retracting, and rotating the hook-shaped electrode 33, in addition to the energizing of a high frequency current. When the conducting wire 37 is advanced and retracted along its own central axial line, it is possible to adjust the protruding length of the hook-shaped electrode 33 from the flexible tube part 31. When the conducting wire 37 is rotated around its own central axial line, the hook-shaped electrode 33 is rotated around the central axial line of the flexible tube part 31, so that it is possible to change the direction of the hook-shaped electrode 33.

The treatment tool 30 of the present embodiment is a medical device capable of incising a biological tissue by connecting a high frequency power supply apparatus to the treatment tool 30 and applying a high frequency current to the biological tissue through the hook-shaped electrode 33.

The structure of the treatment tool 30 is not limited to the medical device using a high frequency current, and a treatment tool having a well-known structure may be appropriately selected and employed as long as it has dimensions by which it is insertable into the treatment tool channel 16. For example, as the treatment tool 30, a snare, a basket, grip forceps, a needle holder, a suture instrument, a test probe or the like for an endoscope may be employed.

Next, the operation and effect of the medical system 1 when used will be described. Fig. 4 is a diagram showing the operation of the medical system 1 when used.

At the time of use of the medical system 1, the external tube 11 of the endoscope device 10 for treatment is inserted into the inside of a body, and the observation device 15 is further inserted into the endoscope channel 12, so that the imaging mechanism 13 is guided up to the vicinity of a site to be treated. The bending operation of the external tube 11, for example, is performed by the master arm 3 by switching a mode of the master arm 3 to a mode of operating the external tube 11 through a foot switch provided on the master manipulator 2.

Furthermore, when it is necessary to treat the site to be treated, the treatment tool 30 is inserted into the treatment tool channel 16 of the external tube 11. Then, the treatment part 32 of the treatment tool 30 is gripped using the grip part 26 provided on the holding tool 20 by operating the grip part provided on the master arm 3. In the present embodiment, any of the two holding tools 20 can grip the treatment part 32.

When the grip member 27 provided on the grip part 26 of the holding tool 20 is opened, fitted to the grooves 36a of the ring part 36, and closed, the treatment part 32 can be held by the grip member 27. At this time, the advance and retraction movement of the ring part 36 to/from the grip member 27 is restricted. Thus, for example, an assistant (not shown) in the vicinity of a patient rotates the conducting wire 37 around the central axial line of the conducting wire 37, so that it is possible to freely change the direction of the hook-shaped electrode 33.

Furthermore, in the case of using the treatment part 32 outside of a movable range of one of the holding tools 20, the treatment part 32 is gripped by the other one of the holding tools 20, so that the treatment part 32 can be used. In the case of switching the treatment part 32 between the two holding tools 20, for example, in the state in which the ring part 36 is gripped in the one of the holding tools 20, a part of the treatment part 32 other than the ring part 36 or the flexible tube part 31 is gripped using the grip part 26 of the other one of the holding tools 20. Thereafter, the one of the holding tools 20 is detached from the ring part 36, and is shifted to a part of the treatment part 32 other than the ring part 36 or the flexible tube part 31. Moreover, the ring part 36 is surrounded by the other one of the holding tools 20. Consequently, the switching of the treatment part 32 and orientation change for a target part of the treatment tool 30 are possible without dropping the treatment part 32.

Furthermore, at the time of the use of the medical system 1, between the two holding tools 20, a holding tool 20 not gripping the treatment part 32 can be used in order to support a tissue X in the vicinity of the site to be treated.

As described above, according to the medical system 1 of the present embodiment, it is possible to simply change the direction, position, orientation or the like of the treatment tool 30 while maintaining the direction of the external tube 11.

Furthermore, in the state in which the movement of the advance and retraction direction of the treatment tool 30 is restricted by the holding tool 20, it is possible to rotate the treatment part 32 by employing the central axial line of the flexible tube part 31 as a rotation center. Consequently, it is possible to adjust the direction of the hook portion 35 to be preferably directed to an object to be treated, and to treat a tissue.

### (Modification 1)

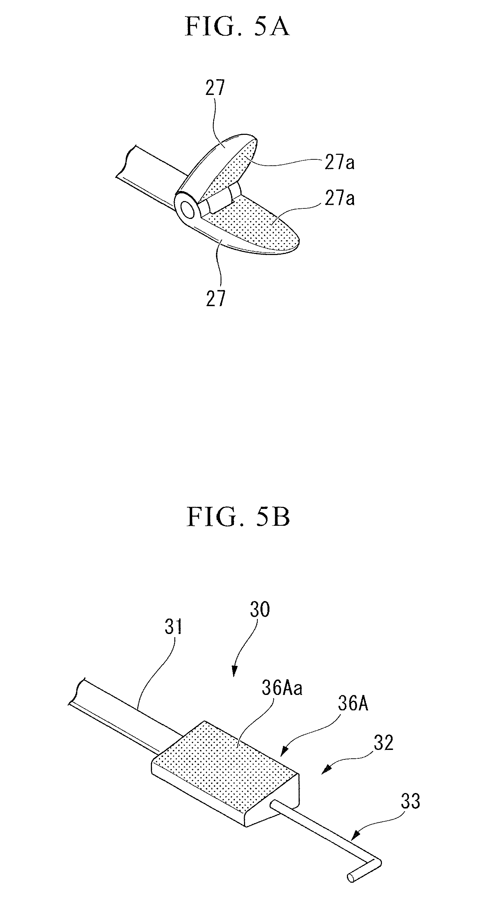

Next, a modification 1 of the aforementioned embodiment will be described. Fig. 5A and Fig. 5B are schematic diagrams showing the structure of the present modification.

As shown in Fig. 5A and Fig. 5B, in the present modification, instead of the ring part 36, a part 36A to be gripped having a frictional surface 36Aa with a concave and convex portion is provided. Furthermore, the grip member 27 is provided with a concave and convex portion 27a so as to frictionally engage with the frictional surface 36Aa.

Even in such a structure, the same effects as those of the aforementioned embodiment are achieved.

### (Modification 2)

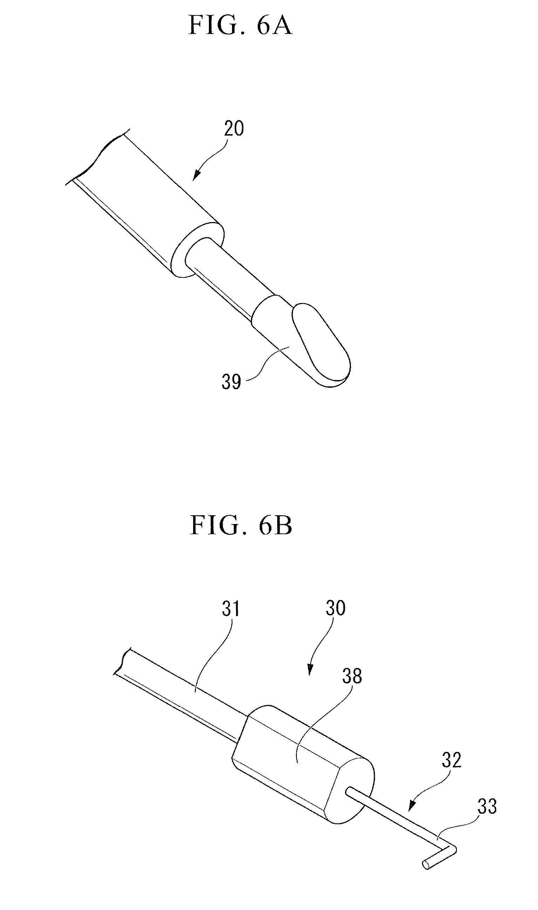

Next, a modification 2 of the aforementioned embodiment will be described. Fig. 6A and Fig. 6B are schematic diagrams showing the structure of the present modification.

As shown in Fig. 6A and Fig. 6B, in the present modification, the treatment part 32 has a body 38 to be absorbed (a part to be held) including a magnetic body, instead of the ring part 36, and the holding tool 20 has an absorbing body 39 having an electromagnet, instead of the grip part 26.

In the present modification, while the electromagnet is being energized, the body 38 to be absorbed can be absorbed by the absorbing body 39. In this case, as compared to the case in which the aforementioned grip part 26 is employed, it is not necessary for an operator to continuously apply force for closing the grip member 27, and it is possible to hold the treatment part 32 through the on-off control of power for the electromagnet.

### (Modification 3)

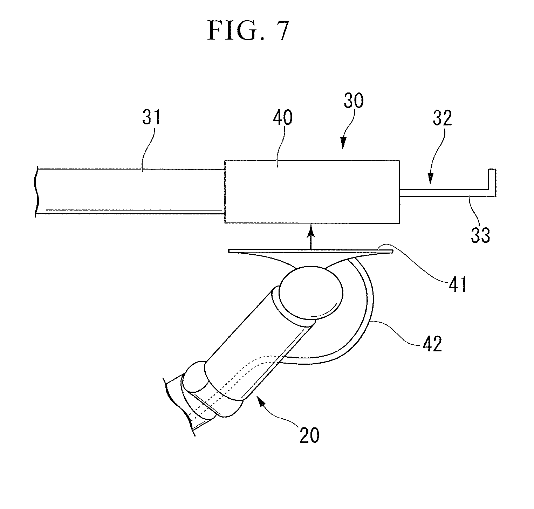

Next, a modification 3 of the aforementioned embodiment will be described. Fig. 7 is a schematic diagram showing the structure of the present modification.

As shown in Fig. 7, in the present modification, the treatment part 32 has a body 40 to be absorbed (a part to be held) including an elastic member such as rubber, instead of the ring part 36, and the holding tool 20 has an absorbing pad 41, instead of the grip part 26.

The absorbing pad 41 is provided with a suction pipeline 42 and decompresses the inside while abutting the body 40 to be absorbed, so that the body 40 to be absorbed is absorbed by a sucker.

Even in such a structure, the same effects as those of the aforementioned embodiment are achieved.

### (Modification 4)

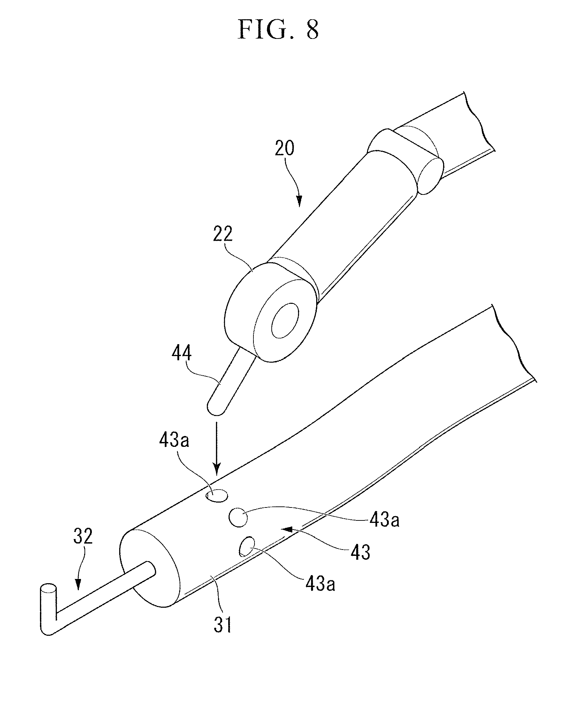

Next, a modification 4 of the aforementioned embodiment will be described. Fig. 8 is a schematic diagram showing the structure of the present modification.

The present modification is different from the aforementioned embodiment in that a part 43 to be inserted (a part to be held) and a rod-shaped part 44 (a holding part) inserted into the part 43 to be inserted are provided as shown in Fig. 8, instead of the ring part 36 and the grip part 26 described in the aforementioned embodiment. In the part 43 to be inserted, holes 43a, into which the rod-shaped part 44 is inserted, are formed in the outer surface of the flexible tube part 31. In the state in which the rod-shaped part 44 is inserted into the part 43 to be inserted, the part 43 to be inserted and the rod-shaped part 44 engage with each other by friction or the like. In the state in which the rod-shaped part 44 is inserted into the part 43 to be inserted, the rod-shaped part 44 is moved, so that the treatment tool 30 having the part 43 to be inserted operates together with the rod-shaped part 44.

In addition, instead of the ring part 36 and the grip part 26 described in the aforementioned embodiment, the rod-shaped part may be provided in a part to be held, and the holes, into which the rod-shaped part is inserted, may be provided in a holding part.

Even in such a structure, the same effects as those of the aforementioned embodiment are achieved.

### (Modification 5)

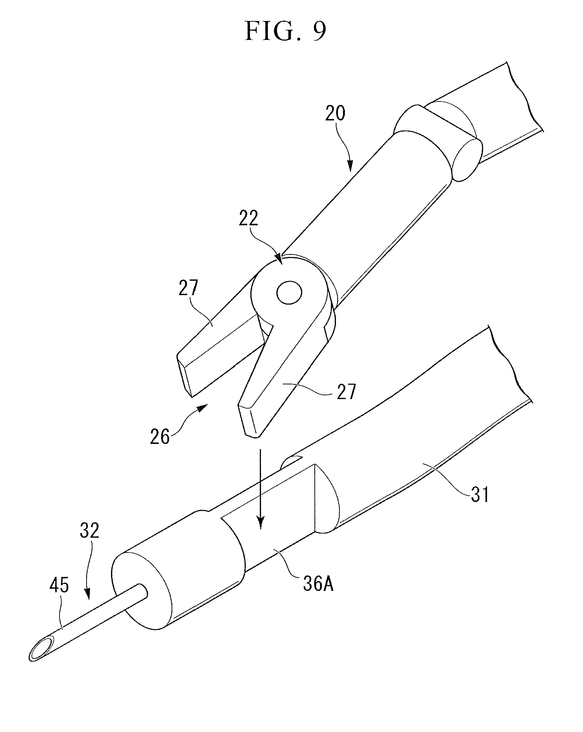

Next, a modification 5 of the aforementioned embodiment will be described. Fig. 9 is a schematic diagram showing the structure of the present modification.

As shown in Fig. 9, in the present modification, a needle tube 45 is disposed at the distal end of the flexible tube part 31 and the inside of the flexible tube part 31 communicates with the inside of the needle tube 45. Consequently, drugs can be injected from the proximal end of the flexible tube part 31 and can be discharged from the distal end of the needle tube 45.

Even in the structure of the present modification, the same effects as those of the aforementioned embodiment are achieved.

### (Modification 6)

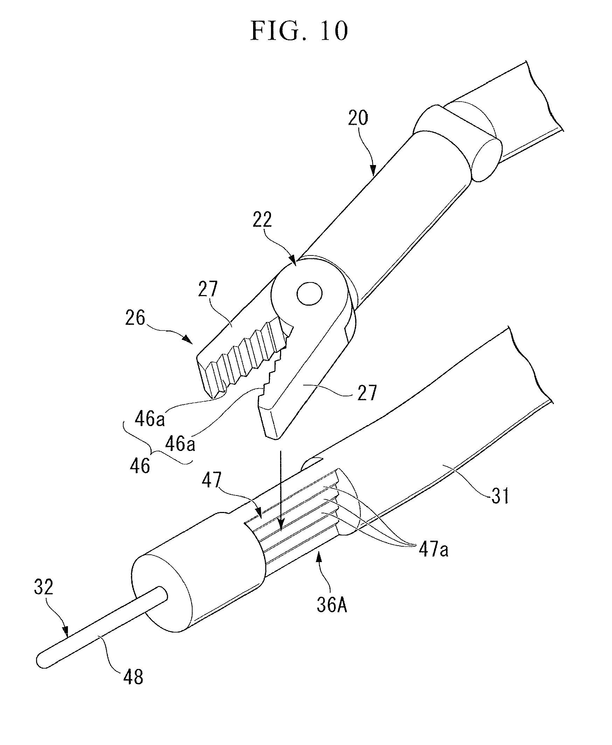

Next, a modification 6 of the aforementioned embodiment will be described. Fig. 10 is a schematic diagram showing the structure of the present modification.

As shown in Fig. 10, in the present modification, the grip part 26 and the part 36A to be gripped have concave-convex shapes 46 and 47 that engage with each other.

The concave-convex shape 46 formed in the grip part 26 is provided on each grip member 27, and has a plurality of projection-stripe portions 46a extending in parallel to one another.

The concave-convex shape 47 formed in the part 36A to be gripped has a plurality of projection-stripe portions 47a extending in parallel to one another on the outer surface of the part 36A to be gripped. The projection-stripe portions 47a extend in the longitudinal axial direction of the flexible tube part 31 (the treatment tool 30).

In addition, in the present modification, as shown in Fig. 10, the plurality of projection-stripe portions 47a extend in the longitudinal axial direction of the flexible tube part 31 (the treatment tool 30); however, the plurality of projection-stripe portions 47a may extend in a direction (an oblique direction) crossing the longitudinal axial direction of the flexible tube part 31 (the treatment tool 30) when viewed from the side of the flexible tube part 31.

Furthermore, in the present modification, a straight needle-shaped electrode 48 provided at the distal end of the flexible tube part 31 is provided as the treatment part 32.

Even in the structure of the present modification, the same effects as those of the aforementioned embodiment are achieved.

### (Modification 7)

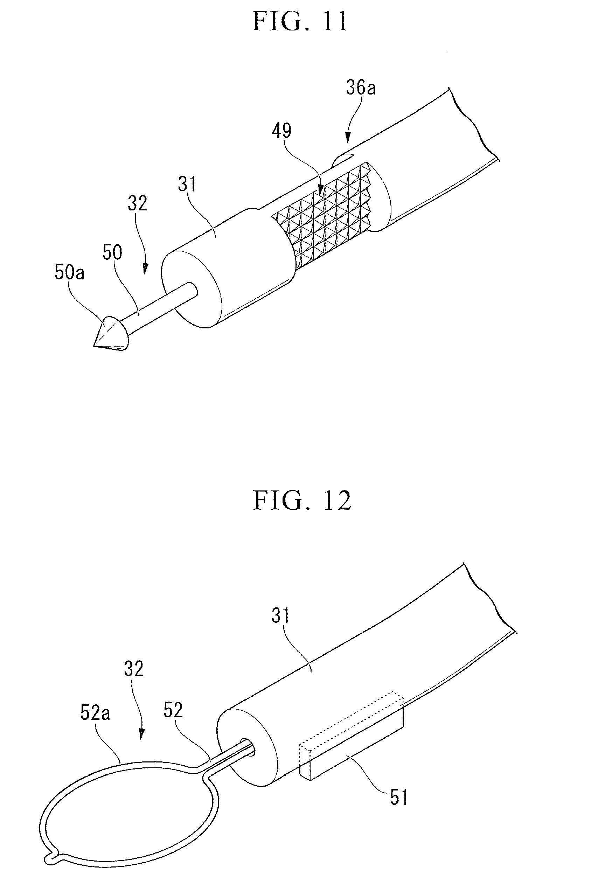

Next, a modification 7 of the aforementioned embodiment will be described. Fig. 11 is a schematic diagram showing the structure of the present modification.

As shown in Fig. 11, in the present modification, the part 36A to be gripped has a plurality of conical-shaped portions 49 having apexes capable of coming in contact with the grip part 26.

Furthermore, in the present modification, as the treatment part 32, a rod-shaped electrode 50 provided with a conical-shaped distal end portion 50a having a diameter that gradually decreases toward the distal end side is provided on the distal end of the flexible tube part 31.

Even in such a structure, the same effects as those of the aforementioned embodiment are achieved.

In addition, it is preferable that the grip surface of the grip part 26 be provided with a plurality of conical-shaped portions that engage with the plurality of conical-shaped portions 49.

### (Modification 8)

Next, a modification 8 of the aforementioned embodiment will be described. Fig. 12 is a schematic diagram showing the structure of the present modification.

As shown in Fig. 12, in the present modification, instead of the part 36A to be gripped, a tab 51 (a part to be held) protruding outward in the diameter direction of the flexible tube part 31 is provided. The tab 51 is gripped by the grip part 26 similarly to the part 36A to be gripped. In this case, the length in the diameter direction of the flexible tube part 31 including the tab 51 is smaller than the inner diameter of the treatment tool channel 16.

Furthermore, in the present modification, as the treatment part 32, a rod-shaped electrode 52 with a snare loop 52a provided on a distal end thereof is provided on the distal end of the flexible tube part 31.

Even in such a structure, the same effects as those of the aforementioned embodiment are achieved.

### (Modification 9)

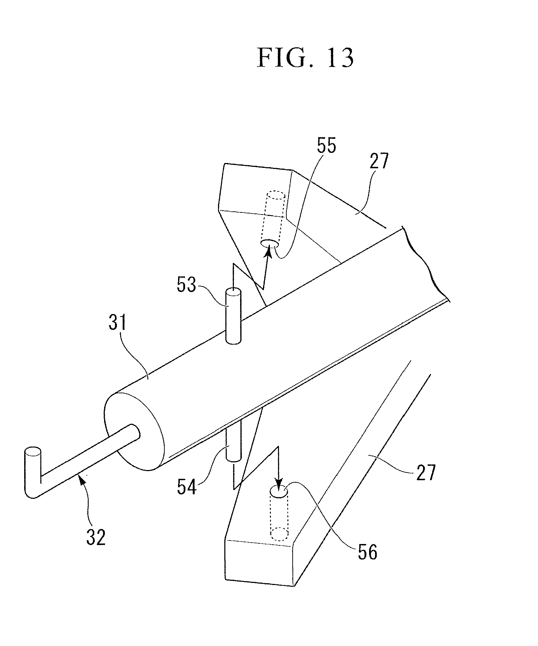

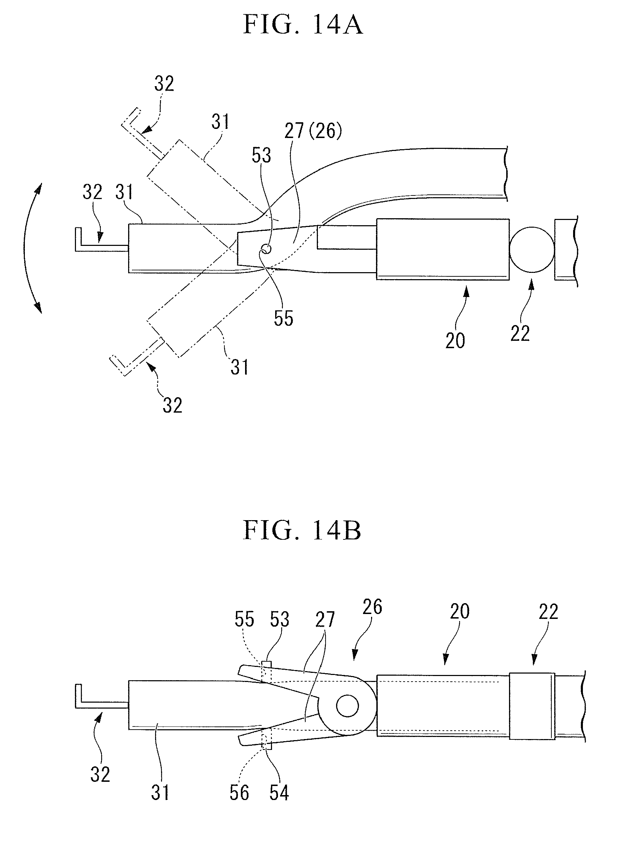

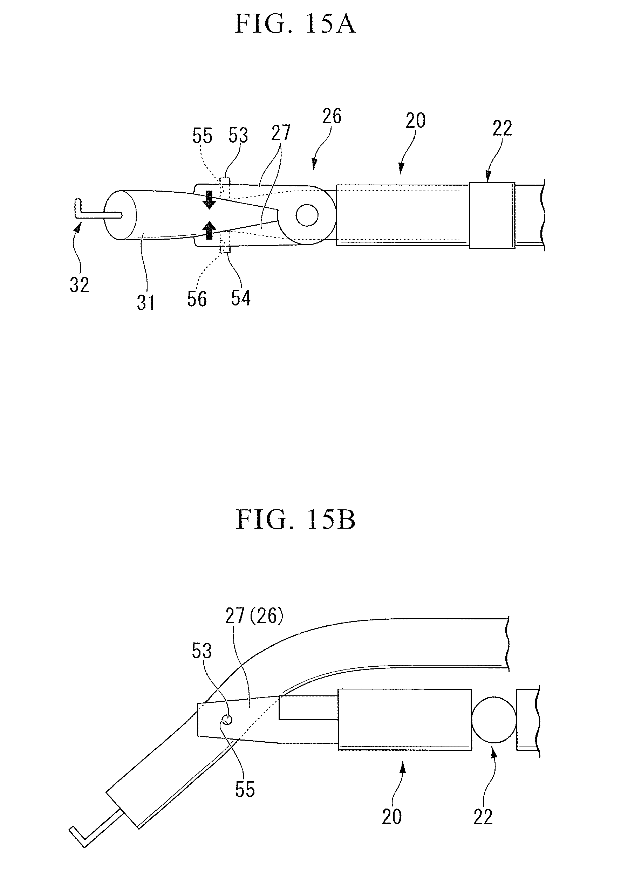

Fig. 13 is a schematic diagram showing the structure of a modification 9 of the aforementioned embodiment. Fig. 14A is a schematic plan view showing an effect of a medical system in the present modification, and Fig. 14B is a schematic side view showing the effect of the medical system in the present modification. Fig. 15A is a schematic side view showing the effect of the medical system in the present modification, and Fig. 15B is a schematic plan view showing the effect of the medical system in the present modification.

As shown in Fig. 13, in the present modification, instead of the part 36A to be gripped, protrusions 53 and 54 (parts to be held) protruding outward in the diameter direction of the flexible tube part 31 are provided. Each of the protrusions 53 and 54 is provided at one of two places which face to the diameter direction of the flexible tube part 31.

Furthermore, holes 55 and 56, into which at least parts of the protrusions 53 and 54 are inserted, respectively, are formed in the grip members 27 of the grip part 26. In this case, the length of the flexible tube part 31 including the protrusions 53 and 54 in the diameter direction is smaller than the inner diameter of the treatment tool channel 16.

In the present modification, in the state in which the protrusions 53 and 54 are inserted into the holes 55 and 56 formed in the grip members 27, the treatment part 32 can be rotated with respect to the grip part 26 by employing the central axial lines of the protrusions 53 and 54 as rotating centers as shown in Fig. 14A. At this time, the treatment part 32 is supported by each grip member 27 so as not to translate in the directions of the central axial lines of the protrusions 53 and 54 as shown in Fig. 14B. Furthermore, as shown in Fig. 15A and Fig. 15B, when the outer surface of the flexible tube part 31 is surrounded by each grip member 27, the direction of the treatment part 32 can be positioned in a desired direction.

So far, the embodiment of the present invention has been described in detail with reference to the accompanying drawings. However, detailed configurations are not limited to the embodiment. For example, a design change and the like within the range without departing from the scope of the present invention are also included.

For example, in the present embodiment, the structure in which the part to be held is held by the holding part by fitting, friction, magnetic force, or absorption has been described; however, the part to be held may be a marking for simply indicating a position at which the treatment part can be preferably held. Such a marking may be configured to inform an operator of desired angle and position at which the treatment part, in which the direction of the hook-shaped electrode when used has been decided, is held.

Furthermore, in the present embodiment, as the operation part, the master arm 3 in the master manipulator 2 of the master-slave type system has been described as an example; however, the present invention is not limited thereto. Even in a structure in which the operation part is directly provided at the distal end of the endoscope device 10 for treatment, the same effects of the present invention are achieved.

Furthermore, in the present embodiment, the structure in which the holding tool is mounted at the distal end of the external tube 11 has been described as an example; however, the holding tool is not limited thereto. For example, a structure in which the holding tool is inserted into a channel formed in the external tube 11 may also be employed. In this case, a portion exposed by the distal end of the external tube 11 is an arm part.

Furthermore, as the treatment tool, a treatment tool that is entirely introduced into the inside of a body and used may also be employed. For example, a knife, a retractor or the like disposed in the inside of the body is employed as the treatment tool, and can be held by the holding tool for use. In this case, the part to be held is provided on the treatment tool itself by performing processing such as shaving of a part other than the treatment part of the treatment tool. As described above, the part to be held is not limited to the part to be held provided on the flexible tube part as in the aforementioned embodiment and each modification.

Furthermore, instead of the treatment tool, a device not for diagnosis or medical cure of the human body can be introduced into the inside of the body, and can be used similarly to the aforementioned embodiment and each modification.

Furthermore, the elements in the aforementioned embodiment and each modification can be appropriately combined with one another.

### Industrial Applicability

According to the medical system and the operation method of the device such as the treatment tool, it is possible to simply change the direction, position, orientation or the like of the device such as the treatment tool while maintaining the direction of the insertion part.

### Reference Signs List

- 1: Medical system
- 3: Master arm
- 10: Endoscope device for treatment
- 11: External tube
- 12: Endoscope channel
- 13: Imaging mechanism
- 14: Illumination mechanism
- 15: Observation device
- 16: Treatment tool channel (Device channel)
- 20: Holding tool
- 21: Tube
- 22: Joint part
- 26: Grip part
- 27: Grip member
- 27a: Concave and convex portion
- 30: Treatment tool
- 31: Flexible tube part
- 32: Treatment part
- 33: Hook-shaped electrode
- 34: Shaft portion
- 35: Hook portion
- 36: Ring part
- 36a: Groove
- 36A: Part to be gripped
- 36Aa: Frictional surface
- 37: Wire
- 38: Body to be absorbed
- 39: Absorbing body
- 40: Body to be absorbed
- 41: Pad
- 42: Suction pipeline
- 43: Part to be inserted
- 43a: Hole
- 44: Rod-shaped part
- 45: Needle tube
- 46: Concave-convex shape
- 46a: Projection stripe
- 47: Concave-convex shape
- 47a: Projection stripe
- 48: Needle-shaped electrode
- 49: Conical-shaped portion
- 50: Rod-shaped electrode
- 50a: Distal end portion
- 51: Tab
- 52: Rod-shaped electrode
- 52a: Snare loop
- 53, 54: Protrusion
- 55, 56: Hole

## Claims

1. A medical system comprising:
an insertion part inserted into an inside of a body;
an arm part provided on the insertion part and capable of moving with respect to the insertion part;
a device disposed in the inside of the body;
a holding part provided on the arm part and holding the device; and
a part to be held provided on the device and held by the holding part.

2. The medical system according to claim 1, wherein at least one of the holding part and the part to be held has a restriction part that restricts movement of the device.

3. The medical system according to claim 1 or 2, wherein the holding part has a grip part that grips the part to be held, and the part to be held has a part to be gripped capable of engaging with the grip part.

4. The medical system according to any one of claims 1 to 3, wherein the holding part and the part to be held have concave-convex shapes engaging with each other.

5. The medical system according to any one of claims 1 to 4, wherein the holding part and the part to be held engage with each other through frictional force.

6. The medical system according to any one of claims 1 to 5, wherein a rod-shaped part is provided on one of the holding part and the part to be held, and a hole to which the rod-shaped part is fitted is formed in a remaining one of the holding part and the part to be held.

7. The medical system according to any one of claims 1 to 6, wherein the part to be held has a plurality of projection-stripe portions, and the plurality of projection-stripe portions extend in parallel to one another in a longitudinal axial direction of the device.

8. The medical system according to any one of claims 1 to 7, wherein the part to be held has a plurality of conical-shaped portions having apexes capable of coming in contact with the holding part.

9. The medical system according to any one of claims 1 to 8, wherein the insertion part has an external tube and a device channel formed in the external tube, and the device has a flexible tube part insertable into the device channel.

10. An operation method of a device disposed in an inside of a body, wherein
a part of the device is held using a holding part provided on an arm part that is the body provided separately from the device and has at least one degree of freedom, and
the arm part is moved, so that the device held by the holding part is moved together with the arm part.
